# EUROPÄISCHE PATENTSCHRIFT

(11) **EP 3 756 745 B1**
(45) Veröffentlichungstag und Bekanntmachung des Hinweises auf die Patenterteilung: **26.05.2021**
(21) Anmeldenummer: 20401039.1
(22) Anmeldetag: 24.06.2020
(51) Int. Cl.: B01D 21/24, B30B 15/04, C02F 11/00, C12M 1/26, C12M 1/00

(54) **VORRICHTUNG ZUR BESCHICKUNG EINER LAGERFLÄCHE**
DEVICE FOR EQUIPPING A SUPPORTING SURFACE
DISPOSITIF DE CHARGEMENT D'UNE SURFACE D'APPUI

(30) Priorität: 25.06.2019 DE 102019117129
(43) Veröffentlichungstag der Anmeldung: 30.12.2020
(73) Patentinhaber: Biogastechnik Süd GmbH, 88316 Isny im Allgäu (DE)
(72) Erfinder: Maier, Clemens, 88316 Isny-Sommersbach (DE); Maier, Gregor, 88316 Isny-Sommersbach (DE)
(74) Vertreter: Höchtl, Walter

(56) Entgegenhaltungen:
- DE-B1- 2 614 588
- US-A1- 2009 314 715

## Beschreibung

Die Erfindung bezieht sich auf eine Vorrichtung zur Beschickung einer Lagerfläche mit einem Separator.

Separatoren werden bei Biogasanlagen, in der Landwirtschaft und Bioabfallwirtschaft zur Trennung von Feststoffen und Flüssigkeiten einer Biomasse, beispielsweise auch von Gülle, verwendet. Der abgetrennte Feststoff kann zur Vergärung in einen Fermenter einer Biogasanlage verbracht werden, oder zur Düngung, zur Gewinnung eines Trockendüngers, oder zur sonstigen Weiterverarbeitung verwendet werden. Der flüssige Anteil wird vorwiegend als Dünger verwendet oder einer sonstigen Weiterverarbeitung zu geführt.

Es sind Separatoren bekannt, die an der Biogasanlage bodennah aufgestellt werden, wobei die Feststoffanteile mittels einer Pumpvorrichtung in einen Fermenter gepumpt werden. Sowohl bei dieser Anwendung als auch dann, wenn die Trennung nicht direkt an der Biogasanlage vorgenommen wird, ist es vorteilhaft, den Separator in einer gewissen Höhe anzubringen, so dass der Feststoffanteil durch sein Eigengewicht auf die Lagerfläche, auf oder in eine Transportvorrichtung oder in einen Gärrestebehälterfallen kann.

Zu diesem Zweck wird der Separator nach dem Stand der Technik (z.B. wie in DE2614588B1 und US2009/314715A1) auf einem Stahlgerüst befestigt. Diese Stahlgerüste sind beispielsweise so ausgeführt, dass sie beidseits oder nur auf einer Seite der Lagerfläche aufbauen und eine Plattform über der Lagerfläche bilden, auf der der Separator befestigt ist. Diese Stahlgerüste sind einerseits sehr kostenintensiv, andererseits relativ immobil, weil sie regelmäßig am Boden befestigt werden müssen und nur mit aufwendiger Demontage an einen anderen Ort verbracht werden können, falls sich der Ort der Lagerfläche verändert. Ein weiterer Nachteil besteht darin, dass für die fest montierten Stahlgerüste eine Baugenehmigung erforderlich ist und sie wegen der vorzusehenden Leitern von der Berufsgenossenschaft abgenommen werden müssen. Außerdem wird regelmäßig ein Anfahrschutz gegen die Beschädigung des Stahlgerüstes benötigt.

Aufgabe der Erfindung ist es deshalb, eine ökonomisch herzustellende Vorrichtung zur Beschickung einer Lagerfläche mit Biomasse mit einem Separator in vorgegebener Höhe zu schaffen, die zudem noch relativ einfach an einen anderen Ort verbracht werden kann und in verschiedener Höhe aufgebaut werden kann.

Diese Aufgabe wird durch die Merkmale des Hauptanspruches gelöst, wobei die Lagerfläche auch ein Transportmittel, beispielsweise ein Container oder die Ladefläche eines Lastkraftwagens, etc. sein kann.

Ein Ausführungsbeispiel der Erfindung wird anhand der Figuren beschrieben. Dabei zeigt die
- Figur 1: eine erfindungsgemäße Vorrichtung in der Vorderansicht,
- Figur 2: eine erfindungsgemäße Vorrichtung in der Seitenansicht

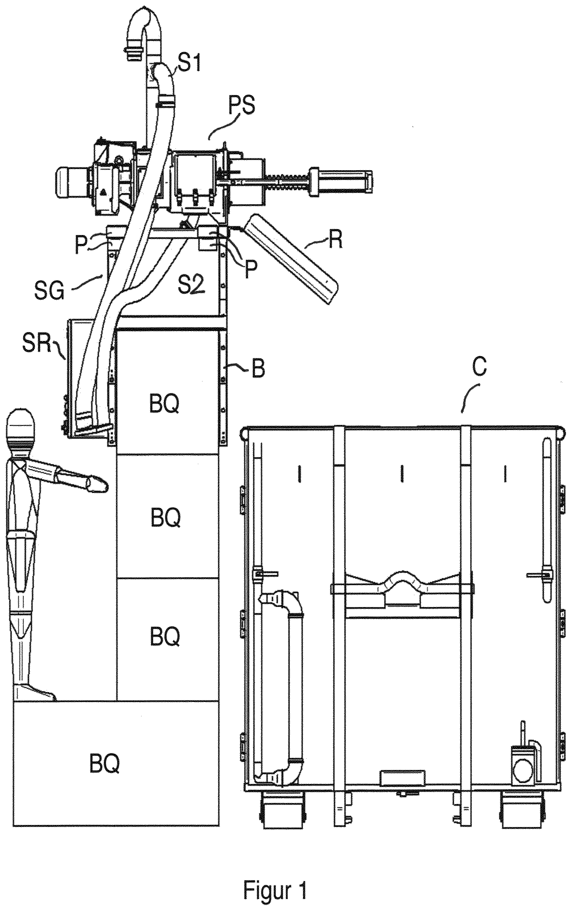

Die Darstellung gemäß Figur 1 zeigt ein Gerüst mit einem Separator PS, wobei der Separator auf einem Stahl- Grundgerüst SG befestigt ist. Das gesamte Gerüst besteht aus dem Stahl-Grundgerüst SG und mehreren Betonquadern BQ. Das Stahl-Grundgerüst SG ist so ausgebildet, dass es mit seinen Beinen B passgenau den obersten Betonquader BQ umfasst. Es kann daran beispielsweise mit Bolzen oder anderen Sicherungsmitteln befestigt werden. In der Regel wird es aber durch sein Eigengewicht und das Gewicht das Separators ohne zusätzliche Befestigungsmittel auf dem oder den Betonquadern BQ halten. Außerdem weist es Gabelstaplertaschen P auf, so dass es mit der Palettengabel eines Gabelstaplers von allen Seiten gegriffen werden kann.

Am Stahl-Grundgerüst SG ist ein Schaltschrank SR befestigt. Sinnvollerweise ist der Schaltschrank SR an den Beinen B des Stahl-Grundgerüstes SG befestigt, so dass er vom Bediener leichter erreichbar ist. Weiter können am Stahl-Grundgerüst SG Befestigungen für die Schlauchstücke S1-S3, nämlich für den Zulaufschlauch für die Biomasse S1, den Ablaufschlauch für den Flüssiganteil S2, und den Überlaufschlauch S3 vorgesehen sein. Damit können die genannten Anschlüsse leicht hergestellt und wieder getrennt werden, wenn das Gerüst an einem anderen Ort aufgestellt werden soll.

Die Betonquader haben beispielsweise Abmessungen von 160x80x40 und/oder 160x80x80. Sie können so ausgestaltet sein, dass ihr Ober- und Unterseiten Rastmittel aufweisen, die beim Aufeinanderstellen verhindern, dass sich die Quader gegeneinander verschieben. Beispielsweise könnten sogenannte Legioblocksteine® verwendet werden. Ein weiteres Beispiel für entsprechende Rastmittel sind Zylinderstumpfverbinungen, Nut/Federverbindungen oder Kegelstumpfverbindungen, wie sie beispielsweise unter https://betonblock-demmar.de/vorteile-von-betonbloecken/demmel-multibloc-system/ gezeigt werden. Insbesondere lässt sich das ebenfalls auf dieser Homepage gezeigte DEMMEL MULTIBLOC-System® verwenden, bei dem In den Betonquadern Sacklöcher vorgesehen sind, in die Rohre so eingebracht werden, dass sie zwei Quader verbinden.

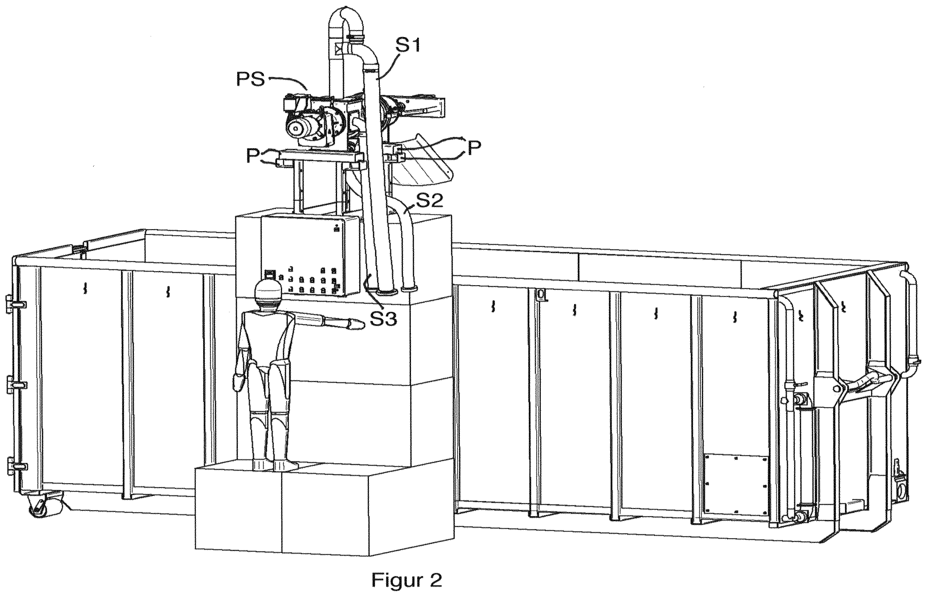

Wie insbesondere in Figur 2 zu sehen, sind die Betonquader BQ so angeordnet, dass sie ein Podest bilden, auf dem eine Bedienperson stehen kann, so dass sie den Schaltschrank und die Schlauchkupplungen erreichen kann. Auf die Wiederholung aller Bezugszeichen in Figur 2 wurde verzichtet, da es sich lediglich um die Seitenansicht handelt.

Die Betonquader können beispielsweise mit einer Gabelstaplerzange gegriffen werden. Es können aber auch entfernbare Ösen vorgesehen werden, so dass am jeweiligen Betonquader BQ Schlaufen zu dessen Handhabung befestigt werden können. Durch den modularen Aufbau des gesamten Gerüstes läßt sich dieses einfach wieder auseinandernehmen und andernorts wieder aufstellen, ohne das aufwendige Demontagearbeiten nötig sind. Auch eine Variation der Höhe der Vorrichtung ist einfach, da nur entsprechend große Betonquader eingefügt oder entnommen werden können. Dazu können die Betonquadermaße auch von den oben genannten Maßen abweichen, indem beispielsweise nur halb- oder drittelhohe oder solche mit beliebig vorgegebener Höhe verwendet werden.

Da die Betonquader eine große Masse aufweisen, ist in der Regel kein Anfahrschutz vorzusehen. Falls ein Betonquader beschädigt wird, ist dieser relativ einfach auswechselbar.

Durch die schiefe Rutsche R am Ausgang des Separators PS, der im Ausführungsbeispiel ein Pressschneckenseparator ist, wird der Feststoffanteil der Biomasse, beispielsweise von Gülle, ausgetragen und fällt in die Lagerfläche, die im Ausführungsbeispiel ein Container C ist, aber auch der Kipper eines LKW's sein kann. Der Feststoffanteil kann auch einfach auf die Lagerfläche, dass heißt auf den Boden unterhalb der Rutsche R fallen. Die Lagerfläche kann dabei vorteilhaft durch eine Mauer oder andere Wand gegenüber den Betonquadern abgetrennt werden.

## Patentansprüche

1. Vorrichtung zur Beschickung einer Lagerfläche mit
1.1 einem Separator (PS)
1.2 einem Stahlgerüst (SG) zur Befestigung des Separators
1.3 mindestens einem Betonquader (BQ), wobei
1.4 der Separator auf dem Stahlgerüst (SG) befestigt ist und das Stahlgerüst (SG) auf einem Betonquader (BQ) derart angeordnet ist, dass die Beine (B) des Stahlgerüstes (SG) mindestens einen Betonquader (BQ) zu beiden Seiten umfassen, wobei an dem Stahlgerüst (SG) ein Schaltschrank (SR) befestigt ist und wobei das Stahlgerüst (SG) Gabelstaplertaschen (P) zur Handhabung mit einer Palettengabel aufweist.

2. Vorrichtung nach Anspruch 1, wobei die Lagerfläche ein Transportmittel (C) ist.

3. Vorrichtung nach einem der vorstehenden Ansprüche, wobei die Betonquader (BQ) so ausgestaltet sind, dass ihre Ober- und Unterseiten ineinandergreifende Rastmittel aufweisen.

## Claims

1. Device for equipping a supporting surface comprising
1.1 a separator (PS)
1.2 a steel frame (SG) for fixing the separator
1.3 at least one concrete block (BQ), wherein
1.4 the separator is fixed on the steel frame (SG) and the steel frame (SG) is arranged on a concrete block (BQ) such that the legs (B) of the steel frame (SG) comprise at least one concrete block (BQ) on both sides, wherein a control cabinet (SR) is fixed on the steel frame (SG) and wherein the steel frame (SG) comprises forklift pockets (P) for handling by means of a pallet fork.

2. Device according to claim 1, wherein the supporting surface is a means of transport (C).

3. Device according to any one of the preceding claims, wherein the concrete blocks (BQ) are configured such that their upper and lower sides comprise interlocking latching means.

## Revendications

1. Dispositif de chargement d'une surface d'appui comportant
1.1 un séparateur (PS)
1.2 une structure en acier (SG) pour la fixation du séparateur
1.3 au moins un cube en béton (BQ), dans lequel
1.4 le séparateur est fixé sur la structure en acier (SG) et la structure en acier (SG) est agencée sur un cube en béton (BQ) de sorte que les pieds de la structure en acier (SG) comportent au moins un cube en béton (BQ) sur les deux côtés, dans lequel une armoire électrique (SR) est fixée à la structure en acier (SG) et dans lequel la structure en acier (SG) comporte des entrées de fourche (P) pour la manipulation avec une fourche à palettes.

2. Dispositif selon la revendication 1, dans lequel la surface d'appui est un moyen de transport (C).

3. Dispositif selon l'une quelconque des revendications précédentes, dans lequel les cubes en béton (BQ) sont configurés de telle sorte que leurs faces supérieures et inférieures comportent des moyens d'arrêt qui s'engrènent.
